# EUROPÄISCHE PATENTANMELDUNG

(11) **EP 2 319 391 A1**
(43) Veröffentlichungstag der Anmeldung: **11.05.2011**
(21) Anmeldenummer: 10190304.5
(22) Anmeldetag: 08.11.2010
(51) Int. Cl.: A61B 1/267

(54) **Beleuchtungsvorrichtung und Bodeneinheit, insbesondere für medizinische Diagnostikinstrumente**

(30) Priorität: 09.11.2009 DE 102009052380; 24.03.2010 DE 202010004123 U
(71) Anmelder: Heine Optotechnik GmbH & Co. KG, 82211 Herrsching (DE)
(72) Erfinder: Heine, Oliver, 82211, Herrsching (DE); Schade, Dirk, 82377, Penzberg (DE); Schneider, Ralf, 80639, München (DE); Plötz, Elsa, 82346, Andechs (DE); Schneider, Anton, 82205, Gilching (DE); Rohrbacher, Bela Michael, 89520, Heidenheim (DE); Atzenbeck, Markus, 86899, Landsberg am Lech (DE)
(74) Vertreter: Hano, Christian

(57) **Zusammenfassung**

Die an der Griffhülse (90) eines Batteriegriffs (11) für medizinische Diagnostikinstrumente (11) anbringbare Beleuchtungsvorrichtung umfasst wenigstens eine LED (36), die über eine Schaltungseinrichtung (50) mit einer Spannungsquelle verbindbar ist, die wahlweise von einer Batterieeinheit (70) oder einer Akkueinheit (74) gebildet wird. Es sind außerdem eine erste Kontakteinrichtung (60) für eine Verbindung der Batterieeinheit (70) und eine zweite Kontakteinrichtung (64) für eine Verbindung der Akkueinheit (74) mit der Schaltungseinrichtung (50) vorgesehen. Eine Bodeneinheit (80) umfasst einen am unteren Ende der Griffhülse (90) anbringbaren Grundkörper (84), in dem ein oberes Kontaktelement (89) für einen Kontakt mit der Akkueinheit (74) und ein unteres Kontaktelement (95) für einen Kontakt mit einem mit Strom beaufschlagten Kontaktelement (107) einer Ladestation angeordnet sind. Zwischen das obere und untere Kontaktelement (89, 95) ist eine Schaltungselektronik (99) mit einem Mikroprozessor (1) geschaltet, die so ausgebildet und konfiguriert ist, dass sie einen Ladevorgang zur Aufladung der Akkueinheit (74) steuert.

## Beschreibung

Die Erfindung betrifft eine Beleuchtungsvorrichtung mit wenigstens einer LED, die über eine Schaltungseinrichtung mit einer Spannungsquelle verbindbar ist, die wahlweise von einer Batterieeinheit oder einer Akkueinheit gebildet wird, sowie eine intelligente Bodeneinheit zur Ladungssteuerung der Akkueinheit. Die Beleuchtungsvorrichtung und die Bodeneinheit sind insbesondere für Handgriffe medizinischer Diagnostikinstrumente, wie z.B. ein Laryngoskop, geeignet.

Aus der DE 203 08 431 U1 ist ein Handgriff für ein Laryngoskop bekannt, der ein zylindrisches längliches Gehäuse aus Kunststoff aufweist, in das ein Lichteinsatz eingesetzt ist, der einen länglichen zylinderförmigen Batteriekörper aufweist. An der Oberseite des Batteriekörpers ist eine Glühlampe vorgesehen, die in ein Einsatzstück aus transparentem Material an dem oberen Ende des Gehäuses vorsteht. Die Glühlampe ist nach oben durch eine Vorspanneinrichtung vorgespannt und in Längsrichtung des Batteriekörpers verschiebbar. In den Batteriekörper kann eine Batterieeinheit bestehend aus zwei Trockenbatterien mit jeweils 1,5 V eingesetzt werden, so dass sich insgesamt eine Spannung von 3 V ergibt. Es ist aber auch möglich, eine Akkueinheit in den Batteriekörper einzuführen, wobei die Spannung eines frisch aufgeladenen Akkus in der Regel 4 V beträgt.

Der Helligkeit, also dem Lichtstrom und der Beleuchtungsstärke der beim Stand der Technik verwendeten Glühlampen, sind technologisch bedingt Grenzen gesetzt. Die Lebensdauer einer Glühlampe beträgt meist wenige Stunden.

Eine mögliche Alternative zur Glühlampe bietet heutzutage zunehmend die LED (Light Emitting Diode)-Technologie. Die Werte für Lichtstrom und Beleuchtungsstärke von LEDs können die Werte von Glühlampen deutlich übersteigen. Darüber hinaus beträgt die zu erwartende Lebensdauer einer LED das Mehrfache einer Glühlampe.

In den heute bereits vorhandenen LED-Anwendungen werden meist Vorwiderstände in Serie zur LED eingesetzt und dann mit einer Spannungsquelle (Batterie oder Akku) verbunden. Die Helligkeit der LED ist somit von der Spannung der Batterie bzw. des Akkus abhängig und schwankt deshalb ungewollt.

Die Versorgung einer LED sollte prinzipiell mit einen konstanten Strom erfolgen, damit eine gleichmäßige Helligkeit gewährleistet ist, und zwar unabhängig von der Batteriespannung. Diesbezüglich ist es denkbar, die LED über einen Stromregler mit einem konstanten Strom zu versorgen, unabhängig von der angelegten Spannung.

Wenn man einem Anwender allerdings die Möglichkeit offen lassen möchte, dass er zwischen Trockenbatterien und Akkus wählen kann, so ergibt sich das Problem, dass für die unterschiedlichen Arten von Spannungsquellen unterschiedliche Arbeitsbereiche zu beachten sind. Eine Trockenbatterie versorgt eine Elektronik mit grundlegend anderen Eingangsspannungswerten als z.B. eine Li-lon-Akkuzelle.

Normalerweise werden bei Laryngoskopen zwei Trockenbatterien in Serie oder eine Akkuzelle als Energieversorgung verwendet. Die beiden Trockenbatterien in Serie haben einen Arbeitsbereich von 2 bis 3 V, während die Akkus einen Arbeitsbereich von 3 bis 4 V aufweisen.

Es ist nicht möglich, einen Stromregler in einem universalen Arbeitsbereich von 2 bis 4 V zu betreiben, weil dies zur Beschädigung der Akkus führen würde. Zum Schutz der Akkus ist es unabdingbar, dass diese nur bis ca. 3 V betrieben werden. Ein Betrieb mit einer Spannung von 2 V würde zu einer irreversiblen Schädigung der Akkuzelle führen.

Bekannte Diagnostikinstrumente, die mit Akkueinheiten betrieben werden, geben weder einen Hinweis auf den Ladezustand der Akkueinheit noch sorgt ein Lademanagement für eine Optimierung des Ladevorgangs. Die Ladezeit wird also nicht optimiert, und der Anwender weiß auch nicht, ob und wenn ja, für wie lange er das Diagnostikinstrument noch benutzen kann, bevor die Akkueinheit wieder aufgeladen werden muss. Dies ist umso problematischer, je älter eine Akkueinheit ist und somit auch unzuverlässig.

Der Erfindung liegt die Aufgabe zugrunde, eine Beleuchtungsvorrichtung mit wenigstens einer LED zu schaffen, die eine gleichmäßige Helligkeit der LED unabhängig von der Batteriespannung gewährleistet, ohne die Funktionsfähigkeit der Batterieeinheit oder der Akkueinheit zu gefährden. Eine weitere Aufgabe besteht darin, eine Bodeneinheit für ein einen Handgriff eines Diagnostikinstrument zu schaffen, durch die eine optimale Ladung der Akkueinheit gewährleistet wird.

Diese Aufgabe wird erfindungsgemäß durch eine Beleuchtungsvorrichtung mit den Merkmalen des Patentanspruchs 1 gelöst. Vorteilhafte Weiterbildungen der erfindungsgemäßen Beleuchtungsvorrichtung sind Gegenstand der Patentansprüche 2 bis 7.

Bei der erfindungsgemäßen Beleuchtungsvorrichtung ist die wenigstens eine LED über eine Schaltungseinrichtung wahlweise mit einer Batterieeinheit oder einer Akkueinheit verbindbar, wobei zwei unterschiedliche Kontakteinrichtungen für eine Verbindung der Batterieeinheit bzw. der Akkueinheit mit der Schaltungseinrichtung vorgesehen sind. Abhängig davon, an welcher Kontakteinrichtung eine Spannung anliegt, kann die Schaltungseinrichtung erkennen, ob als Spannungsquelle eine Batterieeinheit oder eine Akkueinheit verwendet wird.

Bei einer bevorzugten Ausführungsform umfasst die Schaltungseinrichtung eine Schaltungselektronik, die mit der ersten Kontakteinrichtung und mit der zweiten Kontakteinrichtung verbunden ist sowie mit der LED in Verbindung steht. Die Schaltungselektronik ist so ausgebildet und konfiguriert, dass sie einen ersten Betriebsmodus durchführt, wenn die erste Kontakteinrichtung mit der Batterieeinheit verbunden ist, und einen zweiten Betriebsmodus durchführt, wenn die zweite Kontakteinrichtung mit der Akkueinheit verbunden ist. Dadurch, dass die Schaltungselektronik zwei verschiedene Betriebsmodi durchführen kann, ist es möglich, einen Betriebsmodus so durchzuführen, dass er an die Batterieeinheit angepasst ist, und den anderen Betriebsmodus so durchzuführen, dass er für die Akkueinheit optimal ist. Akkueinheit und Batterieeinheit sind gegenseitig austauschbar.

Vorzugsweise wird bei dieser Ausführungsform die Schaltungselektronik so ausgebildet und konfiguriert, dass sie in dem ersten Betriebsmodus eine erste Leistung an der LED anlegt, wenn die Batteriespannung einen ersten Batterie-Schwellwert überschreitet, und keine Leistung an der LED anlegt, wenn die Batteriespannung den ersten Batterie-Schwellwert um ein bestimmtes Maß unterschreitet.

Hierdurch wird es ermöglicht, dass bei Verwendung einer Batterieeinheit eine konstante Leistung an die LED angelegt wird, und dann wenn die Batteriespannung den ersten Batterie-Schwellwert um das bestimmte Maß unterschreitet, die LED ausgeschaltet wird. Hierdurch wird eine Beschädigung der Batterieeinheit vermieden.

Das bestimmte Maß kann auch 0 sein. Dann wird die LED ausgeschaltet, wenn der erste Batterie-Schwellwert unterschritten wird.

Diese Ausführungsform ist vorteilhafterweise so weitergebildet, dass die Schaltungselektronik so ausgebildet und konfiguriert ist, dass sie in dem ersten Betriebsmodus eine zweite konstante Leistung an der LED anlegt, die niedriger als die erste Leistung, wenn die Batteriespannung den ersten Batterie-Schwellwert unterschreitet und höher ist als ein zweiter Batterie-Schwellwert. Hierdurch wird es ermöglicht, dass der Anwender erkennen kann, dass die LED demnächst abgeschaltet wird, wenn das Licht der LED dunkler wird.

Vorzugsweise beträgt der erste Batterie-Schwellwert 2,2 V bis 2,6 V, bevorzugt 2,4 V und der zweite Batterie-Schwellwert 1,8 V bis 2,2 V, bevorzugt 2 V, wenn die Batterieeinheit zwei Trockenbatterien mit einer Nennspannung von je 1,5 V umfasst, die in Reihe geschaltet sind.

Bei einer weiteren bevorzugten Ausführungsform kann die Schaltungselektronik so ausgebildet und konfiguriert sein, dass sie in dem zweiten Betriebsmodus eine erste Leistung an der LED anlegt, wenn die Akkuspannung einen ersten Akku-Schwellwert überschreitet, und keine Leistung an der LED anlegt, wenn die Akkuspannung den ersten Akku-Schwellwert um ein bestimmtes Maß unterschreitet. Durch Abschalten der LED nach Unterschreiten des ersten Akku-Schwellwerts um das vorher bestimmte Maß wird sichergestellt, dass die Akkuspannung nicht so weit abfällt, dass die Akkueinheit beschädigt wird. Wenn die Akkuspannung über dem ersten Akku-Schwellwert liegt, wird die LED mit einer konstanten Leistung betrieben. Auch hier kann das bestimmte Maß auch 0 sein.

Auch in dem zweiten Betriebsmodus kann wie in dem ersten Betriebsmodus eine zweite Leistung an der LED angelegt werden, die niedriger ist als die erste Leistung, wenn die Akkuspannung den ersten Akku-Schwellwert unterschreitet und höher ist als ein zweiter Akku-Schwellwert, der niedriger ist als der erste Akku-Schwellwert, damit der Anwender durch Dunklerwerden des LED-Lichts erkennen kann, dass demnächst die Akkueinheit wieder aufgeladen werden muss, wobei die geringere Helligkeit bei Betrieb mit der zweiten Leistung konstant ist.

In dem zweiten Betriebsmodus beträgt der erste Akku-Schwellwert vorzugsweise 3,2 V bis 3,6 V, bevorzugt 3,4 V, und der zweite Akku-Schwellwert 2,8 V bis 3,2 V, bevorzugt 3,0 V, wenn die voll geladene Akkueinheit eine Spannung von ca. 4 V hat.

Zweckmäßigerweise sind die erste und die zweite Kontakteinrichtung über einen Ein/Aus-Schalter mit der Schaltungselektronik verbunden, um die Spannungszufuhr zwischen der Batterieeinheit bzw. der Akkueinheit zu der Schaltungselektronik zu unterbinden, wenn keine Beleuchtung erforderlich ist.

Zur Einstellung der Helligkeit der LED kann ein Potentiometer vorgesehen sein, durch das die erste Leistung einstellbar ist.

Zur Ladung der Akkueinheit wird erfindungsgemäß eine Bodeneinheit nach Patentanspruch 8 verwendet, die einen am unteren Ende der Griffhülse eines Handgriffs anbringbaren Grundkörper aufweist, in dem ein oberes Kontaktelement für einen Kontakt mit der Akkueinheit und ein unteres Kontaktelement für einen Kontakt mit einem mit Strom beaufschlagten Kontaktelement einer Ladestation angeordnet sind. Zwischen das obere und untere Kontaktelement ist eine Schaltungselektronik mit einem Mikroprozessor geschaltet ist, die so ausgebildet und konfiguriert ist, dass sie einen Ladevorgang zur Aufladung der Akkueinheit steuert.

Die Schaltungselektronik umfasst bevorzugt eine Ladezustandsmesseinrichtung, die so ausgelegt ist, dass sie den momentanen Ladezustands einer Akkueinheit misst und ein entsprechendes Signal an den Mikroprozessor abgibt. In dem Mikroprozessor ist eine Tabelle zur Optimierung der Ladezeit abhängig von dem momentanen Ladezustand der Akkueinheit hinterlegt. Der Mikroprozessor ist so ausgebildet und konfiguriert, dass er einen Laderegler zur Laderegelung der Akkueinheit der abgespeicherten Tabelle entsprechend steuert.

An der Unterseite der Bodeneinheit ist vorzugsweise eine Ladezustandsanzeige vorgesehen, die eine oder mehrere LEDs umfasst. Die Schaltungselektronik ist so ausgebildet und konfiguriert, dass sie den Ladezustand der Akkueinheit über die Ladezustandsanzeige anzeigt, wobei sie bevorzugt die Ladezustandsanzeige abschaltet, wenn das untere Kontaktelement mit dem Kontaktelement der Ladestation in Kontakt kommt, und/oder die Ladezustandsanzeige einschaltet, wenn der Kontakt des unteren Kontaktelements mit dem Kontaktelement der Ladestation unterbrochen wird. Zur Reduzierung des Stromverbrauchs wird die Ladezustandsanzeige vorzugsweise nach Ablauf einer vorher bestimmten Zeit nach dem Einschalten wieder ausschaltet.

Um zu verhindern, dass ein eingeschalteter Verbraucher mehr Strom verbraucht als geladen werden kann, umfasst die Schaltungselektronik zweckmäßigerweise einen Schalter in einer Leiterbahn, die mit dem Verbraucher verbindbar ist, und der Mikroprozessor ist so ausgebildet und konfiguriert, dass er den Schalter öffnet, wenn das untere Kontaktelement mit dem Kontaktelement der Ladestation in Kontakt kommt. Der Schalter wird wieder geschlossen, wenn der Kontakt zwischen dem unteren Kontaktelement und dem Kontaktelement der Ladestation unterbrochen wird.

Die erfindungsgemäße Beleuchtungsvorrichtung und die erfindungsgemäße Bodeneinheit sind vorzugsweise für die Anbringung in einem Laryngoskopgriff geeignet. Es ist aber auch der Einsatz in anderen medizinischen Diagnostikinstrumenten denkbar, bei denen eine Beleuchtung erforderlich ist, wie z.B. in Otoskopen, Dermatoskopen, Ophthalmoskopen oder auch in Handleuchten oder Taschenlampen.

Ein Ausführungsbeispiel der Erfindung wird nachstehend anhand der Zeichnungen näher erläutert. Es zeigen
- Fig. 1: einen Längsquerschnitt durch einen Batteriegriff für ein Laryngoskop im Bereich einer Beleuchtungsvorrichtung, wobei als Spannungsquelle eine Trockenbatterieeinheit verwendet wird,
- Fig. 2: die Ansicht von Fig. 1, wobei als Spannungsquelle eine Akkueinheit verwendet wird,
- Fig. 3: ein Blockschaltbild der Beleuchtungsvorrichtung von Fig. 1,
- Fig. 4: ein Flussdiagramm der Steuerung der Beleuchtungsvorrichtung von Fig. 1 mittels einer ersten Schaltungselektronik,
- Fig. 5: einen Längsquerschnitt durch eine Bodeneinheit des Batteriegriffs,
- Fig. 6: eine Unteransicht der Bodeneinheit von Fig. 5,
- Fig. 7: schematisch den Prinzipaufbau einer Schaltungselektronik der Bodeneinheit von Fig. 5.

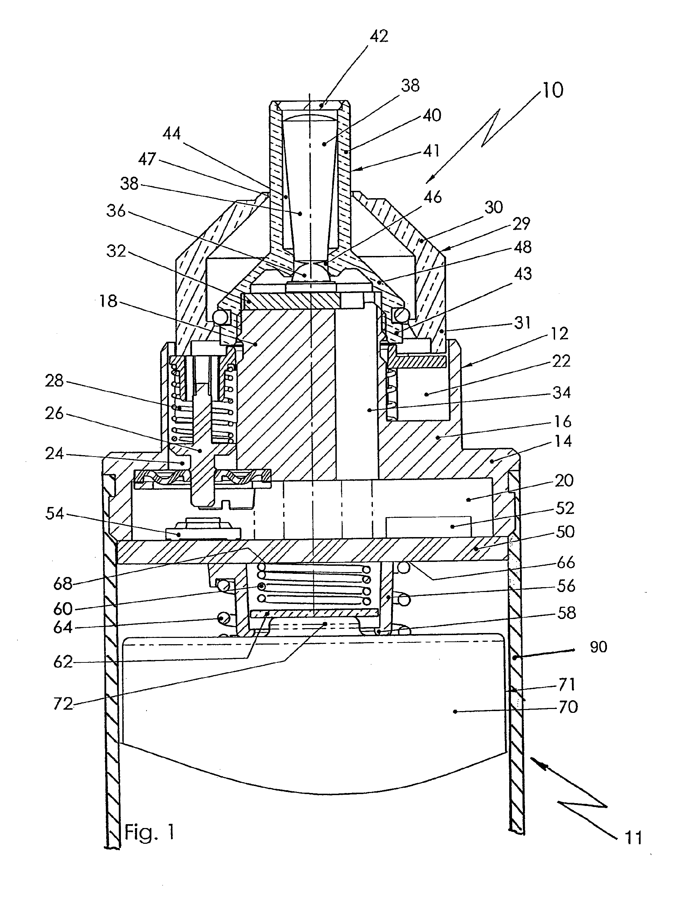

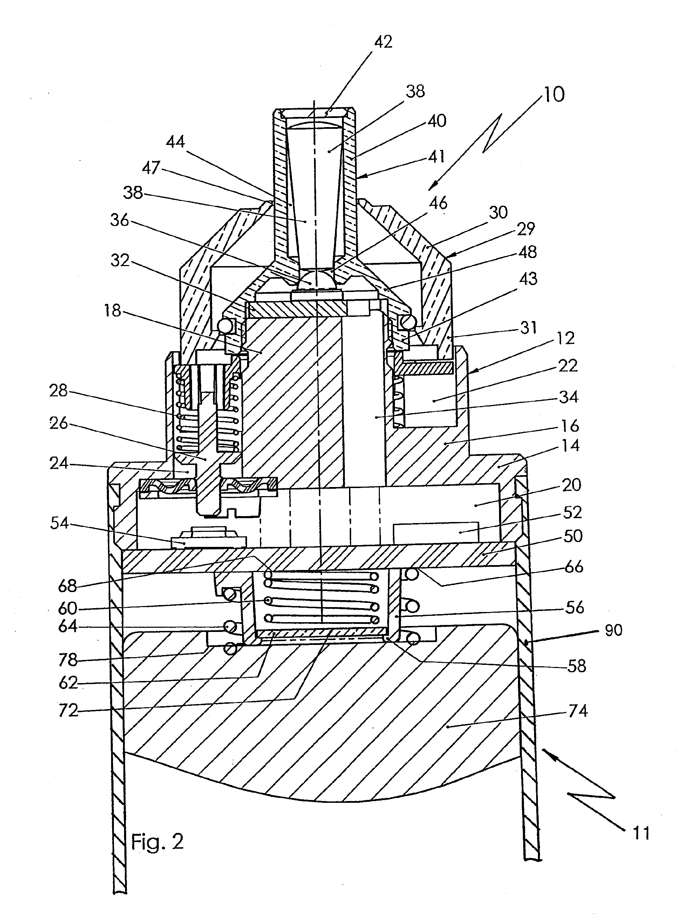

Die in den Figuren 1 und 2 gezeigte Beleuchtungsvorrichtung 10 ist in einen Batteriegriff 11 eines Laryngoskops eingesetzt. Die Beleuchtungsvorrichtung 10 umfasst einen rotationssymmetrischen Grundkörper 12, dessen Außendurchmesser sich nach oben hin stufenförmig verringert, wodurch ein unterer Abschnitt 14 gebildet ist, dessen Außendurchmesser am größten ist und der in das obere Ende einer Griffhülse 90 des Batteriegriffs 11 eingesetzt ist. An diesen unteren Abschnitt 14 schließt sich ein mittlerer Abschnitt 16 an, auf den ein oberer Abschnitt 18 mit geringstem Durchmesser folgt. Über den oberen Abschnitt 18 ist ein Beleuchtungskopf 41 aufgesetzt, der einen untersten zylindrischen Abschnitt 43 umfasst, der den oberen Abschnitt 18 des Grundkörpers 12 umgibt. An den zylindrischen Abschnitt 43 schließt sich ein sich nach oben verjüngender kegelstumpfförmiger Abschnitt 48 an, der in einen oberen zylindrischen Abschnitt 40 übergeht, in dem ein zur Längsachse der Beleuchtungsvorrichtung 10 konzentrischer, zylindrischer Innenraum 44 ausgebildet ist, der an seinem oberen Ende durch eine transparente Abdeckung 42 geschlossen ist. Am unteren Ende des zylindrischen Abschnitts 40 ist eine konzentrische Durchgangsöffnung 46 ausgebildet, deren Durchmesser geringer ist als der Durchmesser des zylindrischen Innenraums 44. In die Durchgangsöffnung 46 erstreckt sich der obere Teil einer LED, an die sich eine Optik 38 anschließt, die sich in Richtung der transparenten Abdeckung 42 erstreckt. Die LED 36 ist mittig auf einer Platine 32 angebracht, die auf der oberen Stirnseite des obersten Abschnitts 18 des Grundkörpers 12 angebracht ist.

An der Unterseite des unteren Abschnitts 14 des Grundkörpers 12 ist eine zylindrische Aussparung 20 ausgebildet, die durch eine Platine 50 abgedeckt ist. Auf der Platine 50 ist eine Schaltungselektronik 52 angebracht. Außerdem ist ein Mikroschalter 54 vorgesehen, der durch einen Schaltstößel 26 betätigbar ist, der parallel zur Längsachse der Beleuchtungsvorrichtung 10 gegen die Kraft einer Schraubenfeder 28 in einer in der Oberseite des mittleren Abschnitts 16 des Grundkörpers 12 ausgebildeten Ringaussparung 22 verschiebbar angeordnet ist und sich auf der in Fig. 1 linken Seite durch eine Durchgangsöffnung 24 nach unten erstreckt, die die Aussparung 20 mit der Ringaussparung 22 verbindet.

Der Beleuchtungskopf 41 wird von einer in Längsrichtung der Beleuchtungsvorrichtung 10 verschiebbaren Druckhülse 29 umgeben, deren unteres Ende in der Ringaussparung 22 angeordnet ist und mittels derer der Schaltstößel 26 betätigt werden kann. An den zylindrischen Abschnitt 31 schließt sich ein sich nach oben verjüngender kegelstumpfförmiger Abschnitt 30 an, der in seinem oberen Ende eine Durchgangsöffnung 47 aufweist, die den zylindrischen Abschnitt 40 des Beleuchtungskopfes 41 auf halber Höhe umschließt.

An der Unterseite der Platine 50 ist konzentrisch eine Hülse 56 aus Isoliermaterial angebracht, die an ihrer unteren Stirnseite einen sich nach innen erstreckenden Bund 58 aufweist. In der Hülse 56 ist eine Kontaktschraubenfeder 60 koaxial angeordnet, die mit ihrem oberen Ende mit einem Kontaktanschluss 68 der Platine verbunden ist. An ihrem unteren Ende ist die Kontaktschraubenfeder 60 mit einer Kontaktplatte 62 verbunden, die aufgrund der Druckkraft der Kontaktschraubenfeder 60 an ihrem Außenumfang auf der Innenseite des Bundes 58 anliegt. Die Hülse 56 wird außerdem von einer zweiten Kontaktschraubenfeder 64 umgeben, deren oberes Ende mit einem Kontaktanschluss 66 auf der Platine 50 verbunden ist und deren unteres Ende auf Höhe des Bundes 58 angeordnet ist, wenn die zweite Kontaktschraubenfeder 64 nicht mit Druck beaufschlagt wird.

Die Kontaktanschlüsse 66, 68 sind jeweils über den Mikroschalter 54 leitend mit der Schaltungselektronik 52 verbunden. Die Schaltungselektronik 52 ist außerdem über eine Plus-Leitung (nicht gezeigt) mit der LED 36 verbunden, die durch eine Durchgangsöffnung 34 in dem Grundkörper 12 geführt ist. Die Minus-Leitung der LED 36 steht elektrisch mit dem Gehäuse des Batteriegriffs in Verbindung.

Fig. 1 zeigt die Verwendung einer Batterieeinheit 71 als Spannungsquelle für die Beleuchtungsvorrichtung 10, die aus zwei in Serie geschalteten und in der Griffhülse 90 angeordneten Trockenbatterien besteht, von denen nur die oberste Trockenbatterie 70 gezeigt ist. Die Trockenbatterie 70 liegt mit ihrer Oberseite an der Stirnseite der Hülse 56 an, wobei sich der zentrale Pluspol 72 durch die Öffnung in dem Bund 58 erstreckt und an der Unterseite der Kontaktplatte 62 anliegt, so dass der Pluspol 72 elektrisch leitend mit der Schaltungselektronik 52 verbunden ist. Der Minuspol wird von dem Gehäuse 90 des Batteriegriffs 11 gebildet.

In Fig. 2 ist die Verwendung einer Akkueinheit 74 als Spannungsquelle gezeigt, die anstatt der Batterieeinheit 71 in der Griffhülse 90 angeordnet ist. Die zylindrische Akkueinheit 74 weist an ihrer Stirnseite eine zentrale zylindrische Aussparung 78 auf, deren Durchmesser größer ist als der Außendurchmesser der Hülse 56. Die zweite Kontaktschraubenfeder 64, die die Hülse 56 umgibt, drückt auf den Boden der Aussparung 78, wodurch eine leitende Verbindung des Pluspols der Akkueinheit 74 mit der Schaltungselektronik 52 gebildet wird. Der Boden der Aussparung 78 liegt an der Stirnseite der Hülse 56 an.

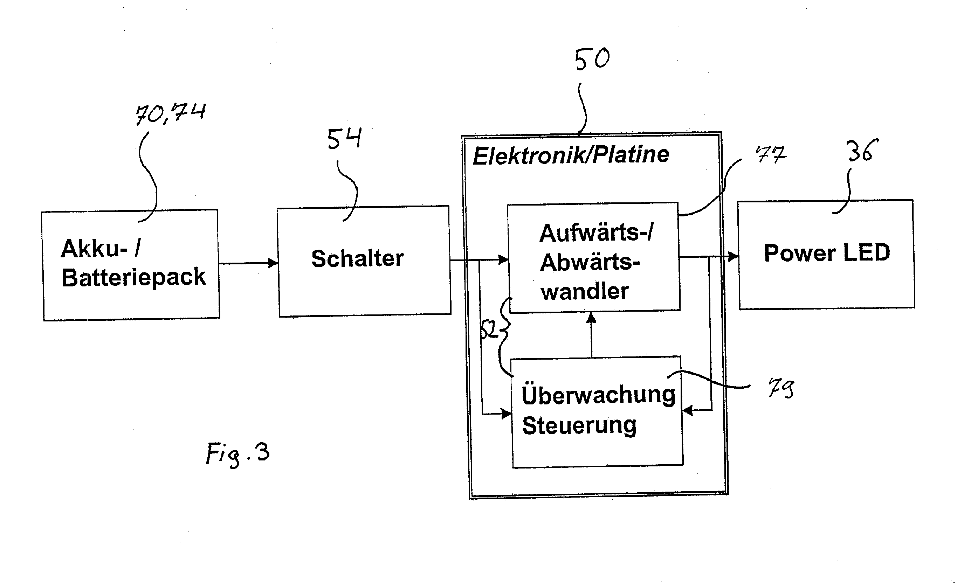

In Fig. 3 ist ein Blockschaubild der Leitungsverbindung zwischen dem Akku/Batterie-Pack 70, 74 und der LED 36 gezeigt. Zwischen der Schaltungselektronik 52 auf der Platine 50 ist der Mikroschalter 54 angeordnet, durch den die leitende Verbindung zwischen Akku/Batterie-Pack 70, 74 und der Schaltungselektronik 52 unterbrochen und wiederhergestellt werden kann.

Die Schaltungselektronik 52 umfasst eine Steuer- und Überwachungseinheit 79 sowie einen Aufwärts-/Abwärts-Wandler 77, der von der Steuer- und Überwachungseinheit 79 gesteuert wird und Leistung in zwei unterschiedlichen Stufen an die LED 36 anlegen kann, wobei die Leistung der niedrigen Leistungsstufe beispielsweise 100 mA und die Leistung der hohen Leistungsstufe beispielsweise 350 mA betragen kann. Die LED wird in diesem Fall konstant mit einer Spannung von 3 V betrieben.

Die Schaltungselektronik 52 ist so konfiguriert und ausgelegt, dass sie zwei Betriebsmodi durchführen kann, nämlich einen ersten Betriebsmodus für den Fall, dass als Spannungsquelle eine Batterieeinheit 71 verwendet wird, und einen zweiten Betriebsmodus im Falle der Verwendung einer Akkueinheit 74 als Spannungsquelle. Die Schaltungselektronik 52 ist dabei so ausgebildet und konfiguriert, dass sie dafür sorgt, dass eine Abschaltung erfolgt, sobald die Batterie- oder Akkuspannung einen unteren Schwellwert erreicht, dessen Unterschreitung zu einer Beschädigung der Batterieeinheit oder des Akkus führen würde. Solange die Batterie- oder Akkuspannung einen oberen Schwellwert nicht unterschreitet, wird über den Aufwärts-/Abwärts-Wandler 77 die LED mit der Leistung der hohen Leistungsstufe betrieben, so dass sie eine konstante hohe Helligkeit aufweist. Wenn die Spannung der Batterieeinheit bzw. der Akkueinheit den oberen Schwellwert unterschreitet aber höher ist als der untere Schwellwert, wird von der Steuer- und Überwachungseinheit 80 der Schaltungselektronik 52 ein entsprechendes Signal an den Aufwärts-/Abwärts-Wandler 77 abgegeben, woraufhin dieser die niedrige Leistungsstufe für die LED 36 wählt, so dass die LED 36 mit einer geringeren, aber konstanten Helligkeit leuchtet, bis die Spannung unter den unteren Schwellwert fällt. Dann wird die Beleuchtung ausgeschaltet.

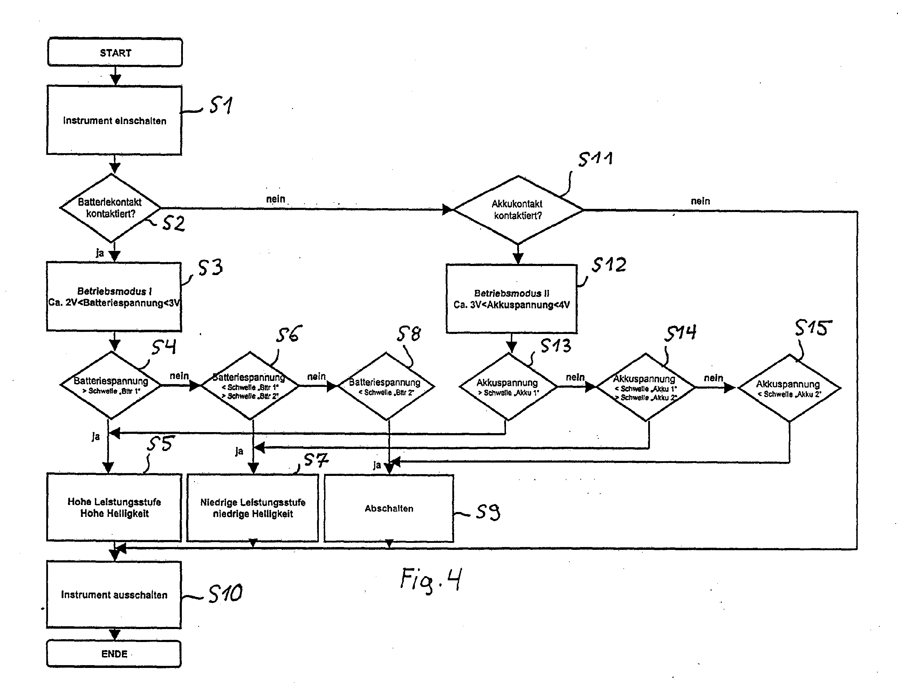

Ein entsprechendes Flussdiagramm ist in Fig. 4 gezeigt und wird im Folgenden beschrieben. Zunächst wird das Instrument durch Betätigung des Mikroschalters 54 eingeschaltet (Schritt S1). Die Schaltungselektronik 52 überprüft dann, ob an dem Kontaktanschluss 68 eine Spannung anliegt. Wenn ja, bestimmt die Schaltungselektronik 52, dass der Batteriekontakt kontaktiert ist (Schritt S2) und wählt einen ersten Betriebsmodus, in dem die Batteriespannung zwischen 2 V und 3 V liegen soll (Schritt S3). Anschließend wird bestimmt, ob die Batteriespannung einen ersten Schwellwert Bttr1 überschreitet (Schritt S4). Wenn ja, wählt die Schaltungselektronik 52 über den Aufwärts-/Abwärts-Wandler 77 eine hohe Leistungsstufe, so dass eine konstante Leistung mit beispielsweise 350 mA an der LED angelegt wird (Schritt S5). Wenn die Schaltungselektronik 52 feststellt, dass die Batteriespannung unterhalb des Schwellwerts Bttr1, jedoch oberhalb eines zweiten Schwellwerts Bttr2 liegt (Schritt S6), wird der Aufwärts-/Abwärts-Wandler 77 von der Steuer- und Überwachungseinheit 79 so angesteuert, dass er die niedrige Leistungsstufe einstellt, in der die LED 36 mit einer konstanten Leistung von beispielsweise 100 mA betrieben wird (Schritt S7).

Wenn jedoch festgestellt wird, dass die Batteriespannung auch den zweiten Schwellwert Bttr2 unterschreitet (Schritt S8), wird die Stromversorgung der LED 36 abgeschaltet (Schritt S9).

Wenn nach Einschalten des Instruments (Schritt S1) in dem Schritt S2 festgestellt wird, dass der Batteriekontakt 68 nicht kontaktiert ist, überprüft die Schaltungselektronik 52, ob der Akkukontakt 66 kontaktiert ist (Schritt S11). Wenn dies der Fall ist, wird ein zweiter Betriebsmodus in einem Schritt S12 gewählt, in dem der erlaubte Spannungsbereich zwischen ca. 3 V und 4 V liegt. Die Schaltungselektronik überprüft anschließend, ob die Akkuspannung oberhalb eines Schwellwerts Akku1 liegt (Schritt S13). Wenn ja, wird zu Schritt S5 übergegangen, das heißt, es wird die hohe Leistungsstufe für die LED 36 gewählt. Wenn die Akkuspannung zwischen dem oberen Schwellwert Akku1 und einem zweiten unteren Schwellwert Akku2 liegt (Schritt S14), wird der Schritt S7 gewählt, das heißt, es wird die niedrige Leistungsstufe für die LED 36 gewählt. Liegt die Akkuspannung jedoch auch unter dem zweiten Schwellwert Akku2 (Schritt S15), wird die LED 36 in dem Schritt S9 abgeschaltet.

Die Überprüfung der Spannungen findet in dem ersten und zweiten Betriebsmodus fortlaufend statt, bis das Instrument in dem Schritt S10 ausgeschaltet wird.

Bei der oben beschriebenen Ausführungsform umfasst die Batterieeinheit zwei Trockenbatterien mit einer Nennspannung von je 1,5 V und die Akkueinheit hat eine Nennspannung von 4 V. Abhängig von der Betriebsspannung der eingesetzten LED können aber auch Akkueinheiten oder Batterieeinheiten mit höherer oder niedriger Nennspannung eingesetzt werden. Die jeweiligen zweiten Schwellwerte müssen dann so gesetzt werden, dass eine Beschädigung der Akkueinheiten oder Batterieeinheiten vermieden wird. Der erste Schwellwert muss dann entsprechend angepasst werden und liegt bevorzugt 0,4 V über dem zweiten Schwellwert.

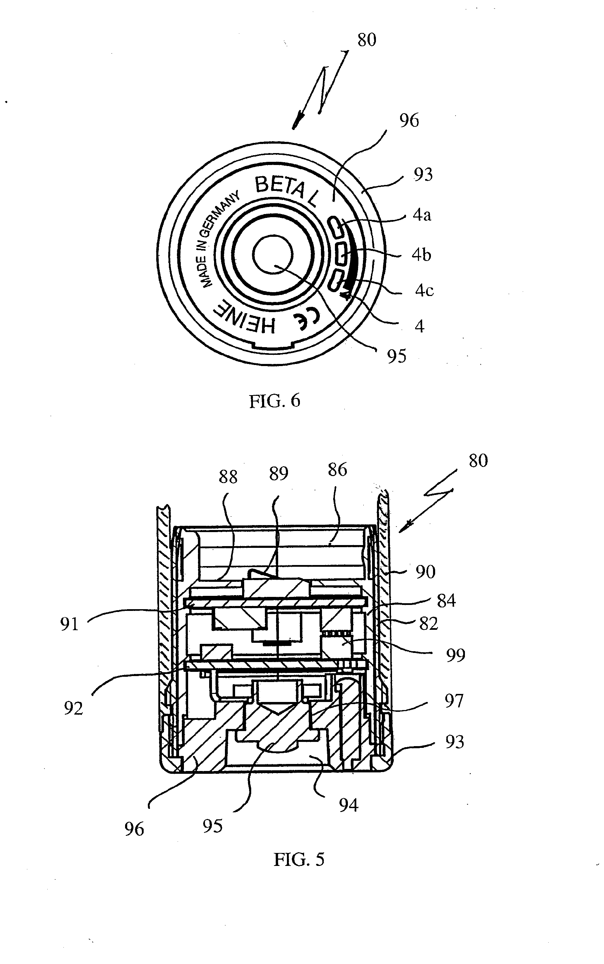

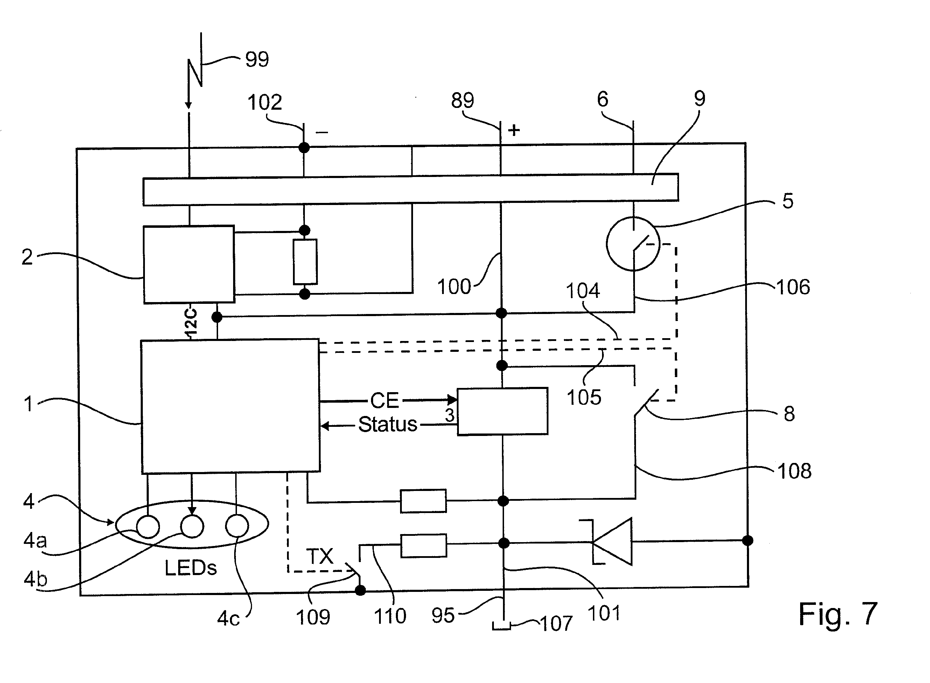

In den Figuren 5 und 6 ist eine Bodeneinheit 80 gezeigt, die an dem unteren Ende der Griffhülse 90 angebracht ist. Die Bodeneinheit 80 umfasst einen rotationssymmetrischen Grundkörper 84 aus Kunststoff der von einer Metallhülse 84 umgeben ist, deren Außendurchmesser im Wesentlichen dem Innendurchmesser der Griffhülse 90 entspricht und die in das untere Ende der Griffhülse 90 eingeschoben ist. In der oberen Stirnseite des Grundkörpers 84 ist eine Vertiefung 86 zur Aufnahme des unteren Endes einer Akkueinheit 74 (Figur 2) ausgebildet. An seinem unteren Ende ist der Grundkörper 84 durch ein Bodenstück 96 aus Kunststoff verschlossen, das durch einen auf das untere Ende der Metallhülse 82 aufgeschraubten Metallring 93 gehalten ist, dessen obere Stirnseite an dem unteren Ende der Griffhülse 90 anliegt, die ebenfalls aus Metall gebildet ist. Zwischen dem Boden 88 der Vertiefung 86 und dem Bodenstück 96 sind innerhalb des Grundkörpers 84 zwei Schaltungsplatinen 91, 92 im Abstand zueinander so angeordnet, dass ihre Ebene senkrecht zur Mittelachse des Grundkörpers 84 verläuft. Auf den Schaltungsplatinen 91, 92 ist eine Schaltungselektronik 99 angeordnet, deren Ausbildung nachstehend anhand der Figur 7 näher erläutert wird.

Durch den Boden 88 der Vertiefung 86 erstreckt sich von unten ein Kontaktelement 89, durch das eine elektrische Verbindung der Schaltungselektronik 99 mit dem Pluspol der Akkueinheit 74 hergestellt wird. In dem Bodenstück 96 ist zentral eine Durchgangsöffnung 97 anschließend an eine Vertiefung 94 in der unteren Stirnseite des Bodenstücks 96 ausgebildet. In der Durchgangsöffnung 97 ist ein Kontaktelement 95 angeordnet, dessen unteres Ende in der Vertiefung 94 endet und dessen oberes Ende mit einer Plus-Leiterbahn der Schaltungsplatine 99 elektrisch verbunden ist. Der Minuskontakt wird von dem Metallring 93 und der Metallhülse 82 gebildet.

Der Prinzipaufbau der Schaltungselektronik 99 ist in Figur 7 gezeigt. Die Schaltungselektronik 99 weist als zentrales Element einen Mikroprozessor 1 auf. Außerdem ist eine Ladezustandsmesseinrichtung 2 vorgesehen, die einerseits mit einem Minuskontakt 102 und andererseits über eine Leiterbahn 100 mit dem Kontaktelement 89 in Verbindung steht, das den Pluspol der Akkueinheit 74 kontaktiert. Die Ladezustandsmesseinrichtung 2 ermittelt den Ladezustand der Akkueinheit 74 und gibt ein entsprechendes Signal an den Mikroprozessor 1 weiter. Außerdem ist eine LED-Einrichtung 4 mit drei LEDs 4a, 4b, 4c unterschiedlicher Farbe vorgesehen, die von dem Mikroprozessor 1 dem jeweiligen Ladezustand entsprechend aktiviert werden.

In dem Mikroprozessor 1 ist eine Tabelle hinterlegt, aus der der Mikroprozessor 1 die jeweils optimale Ladespannung für einen bestimmten gemessenen Ladezustand liest. Diese Ladespannung gibt der Mikroprozessor 1 an einen Laderegler 3 weiter, der über die Leiterbahn 100 mit dem Kontaktelement 89 verbunden ist. Ein anderer Ausgang des Ladereglers 3 steht über eine Leiterbahn 101 mit dem Kontaktelement 95 in Verbindung. Die Leiterbahn 100 ist darüber hinaus über eine Leiterbahn 106 mit einem Verbraucher 6, z.B. mit der Plusleitung der Beleuchtungsvorrichtung 10, verbunden, wobei in der Leiterbahn 106 ein Schalter 5 angeordnet ist, der von dem Mikroprozessor 1 über eine Leitung 104 gesteuert wird.

Die Leiterbahn 100 und die Leiterbahn 101 sind über eine Leiterbahn 108 verbindbar, die den Laderegler 3 umgeht. In der Leiterbahn 108 ist ein Schalter 8 angeordnet, der ebenfalls von dem Mikroprozessor 1 gesteuert wird. Wenn der Schalter 8 geschlossen ist, wird eine direkte Verbindung zwischen den Leiterbahnen 100 und 101 und somit zwischen dem Kontaktelement 95 und dem Kontaktelement 89 hergestellt. Schließlich ist noch ein ESD-Element 9 vorgesehen, das die Elemente der Schaltungselektronik 99 vor elektrostatischer Ladung schützt.

Wenn der Batteriegriff 11 in eine Ladestation gesteckt wird und ein Kontakt zwischen einem mit Strom beaufschlagten Kontaktelement 107 der Ladestation und dem Kontaktelement 95 hergestellt wird, tauscht der Mikroprozessor 1 über eine Leitung 110, in der ein Schalter 109 angeordnet ist, ein entsprechendes Kommunikationsprotokoll aus und stellt dann die Verbindung zwischen der Ladestation und der Bodeneinheit 80 her. Der Ladezustand der Akkueinheit 74 wird über die Ladezustandsmesseinrichtung 2 ermittelt und an den Mikroprozessor 1 weitergegeben. Durch das gezielte Ermitteln des aktuellen Ladezustands durch die Ladezustandsmesseinrichtung 2 ermittelt der Mikroprozessor 1 anhand der abgespeicherten Tabelle die Werte zur Optimierung der Ladezeit und steuert den Laderegler 3 entsprechend. Die damit verbundenen unterschiedlichen Ladeströme zur Akkueinheit 74 über das Kontaktelement 89 werden durch den Laderegler 3 gesteuert und in die Akkueinheit 74 eingespeist. Es wird zwischen Schnellladung, Ladung oder Ladungserhaltung unterschieden.

Wenn der Austausch des Kommunikationsprotokolls zwischen Mikroprozessor 1 und Ladestation nicht erfolgreich ist, wird der Schalter 8 geschlossen.

Wenn der Batteriegriff 11 wieder der Ladestation entnommen wird, wird der Kontakt zwischen den Kontaktelementen 95 und 107 unterbrochen und es wird der aktuelle Ladezustand mittels einer oder mehrerer der LEDs 4a, 4b, 4c der LED-Einrichtung angezeigt, wobei die LEDs 4a, 4b, 4c durch den Mikroprozessor 1 entsprechend gesteuert werden. Der Mikroprozessor 1 steuert die LED-Einrichtung 4 darüber hinaus so, dass die LEDs 4a, 4b, 4c nach einer vorherbestimmten Zeit zur Schonung der Akkueinheit 74 wieder ausgeschaltet werden.

Der Mikroprozessor 1 aktiviert die Ladezustandsanzeige 4 auch dann, wenn der Griff nicht direkt der Ladestation entnommen wird, sondern im entnommenen Zustand der Verbraucher 6 zugeschaltet wird.

Je nach verwendetem Verbraucher 6 ist es möglich, dass bei eingeschaltetem Verbraucher mehr Strom verbraucht wird, als geladen werden kann. Damit die Akkueinheit 74 nicht weiter entladen wird, obwohl sie eigentlich geladen werden soll, und zur Schonung der Lampe in z.B. der Beleuchtungseinrichtung 10 prüft der Mikroprozessor 1 nach Einstecken des Batteriegriffs 11 in die Ladestation, ob der Verbraucher 6 ein- oder ausgeschaltet ist. Ist der Verbraucher 6 nicht ausgeschaltet, so schaltet der Mikroprozessor den Schalter 5 so, dass die Stromversorgung des Verbrauchers 6 über die Leiterbahn 106 unterbrochen wird. Wenn der Batteriegriff wieder der Ladestation entnommen wird, wird der Schalter 5 wieder geschlossen, so dass der Verbraucher 6 mit Strom versorgt wird.

Wenn der Mikroprozessor 1 bei Einstecken des Batteriegriffs 11 in eine Ladestation erkennt, dass eine Laderegelung bereits in der Ladestation vorgesehen ist, schließt der Mikroprozessor 1 den Schalter 8, wodurch ein direkter Kontakt der Kontaktelemente 89, 95 über die Leiterbahnen 101,108 und 100 hergestellt wird und eine Laderegelung der Schaltungselektronik 99 unterbunden wird.

## Patentansprüche

1. Beleuchtungsvorrichtung, insbesondere für medizinische Diagnostikinstrumente, mit wenigstens einer LED (36), die über eine Schaltungseinrichtung (50) mit einer Spannungsquelle verbindbar ist, die wahlweise von einer Batterieeinheit (70) oder einer Akkueinheit (74) gebildet wird, die gegenseitig austauschbar sind, wobei eine erste Kontakteinrichtung (60) für eine Verbindung der Batterieeinheit (70) und eine zweite Kontakteinrichtung (64) für eine Verbindung der Akkueinheit (74) mit der Schaltungseinrichtung (50) vorgesehen sind.

2. Beleuchtungsvorrichtung nach Anspruch 1, **dadurch gekennzeichnet, dass** die Schaltungseinrichtung eine Schaltungselektronik (52) umfasst, die mit der ersten Kontakteinrichtung (60) und mit der zweiten Kontakteinrichtung (64) verbunden ist sowie mit der LED (36) in Verbindung steht, wobei die Schaltungselektronik (52) so ausgebildet und konfiguriert ist, dass sie einen ersten Betriebsmodus durchführt, wenn die erste Kontakteinrichtung (60) mit der Batterieeinheit (70) verbunden ist, und einen zweiten Betriebsmodus durchführt, wenn die zweite Kontakteinrichtung (64) mit der Akkueinheit (74) verbunden ist.

3. Beleuchtungsvorrichtung nach Anspruch 2, **dadurch gekennzeichnet, dass** die Schaltungselektronik (52) so ausgebildet und konfiguriert ist, dass sie in dem ersten oder zweiten Betriebsmodus
- eine erste Leistung an der LED (36) anlegt, wenn die Batteriespannung bzw. die Akkuspannung einen ersten Schwellwert überschreitet, und
- keine Leistung an der LED (36) anlegt, wenn die Batteriespannung bzw. die Akkuspannung den ersten Schwellwert um ein bestimmtes Maß unterschreitet.

4. Beleuchtungsvorrichtung nach Anspruch 3, **dadurch gekennzeichnet, dass** der erste Schwellwert der Batteriespannung 2,2 V bis 2,6 V und der erste Schwellwert der Akkuspannung 3,2 V bis 3,6 V beträgt.

5. Beleuchtungsvorrichtung nach Anspruch 2, **dadurch gekennzeichnet, dass** die Schaltungselektronik (52) so ausgebildet und konfiguriert ist, dass sie in dem ersten oder zweiten Betriebsmodus
- eine erste Leistung an der LED (36) anlegt, wenn die Batteriespannung bzw. die Akkuspannung einen ersten Schwellwert überschreitet, und
- eine zweite Leistung an der LED (36) anlegt, die niedriger ist als die erste Leistung, wenn die Batteriespannung bzw. die Akkuspannung den ersten Schwellwert unterschreitet und höher ist als ein zweiter Schwellwert, der niedriger ist als der erste Schwellwert.

6. Beleuchtungsvorrichtung nach Anspruch 5, **dadurch gekennzeichnet, dass** der zweite Schwellwert der Batteriespannung 1,8 V bis 2,2 V und der zweite Schwellwert der Akkuspannung 2,8 V bis 3,2 V beträgt.

7. Batteriegriff für medizinische Diagnostikinstrumente, insbesondere für ein Laryngoskop, mit einer Beleuchtungsvorrichtung nach einem der vorhergehenden Ansprüche.

8. Bodeneinheit für einen Batteriegriff (11) für medizinische Diagnostikinstrumente, der eine Griffhülse (90) umfasst, in der eine Akkueinheit (74) angeordnet ist, mit der ein Verbraucher (36) einer Beleuchtungseinrichtung (10) in Verbindung bringbar ist, mit einem am unteren Ende der Griffhülse (90) anbringbaren Grundkörper (84), in dem ein oberes Kontaktelement (89) für einen Kontakt mit der Akkueinheit (74) und ein unteres Kontaktelement (95) für einen Kontakt mit einem mit Strom beaufschlagten Kontaktelement (107) einer Ladestation angeordnet sind, **dadurch gekennzeichnet, dass** zwischen das obere und untere Kontaktelement (89, 95) eine Schaltungselektronik (99) mit einem Mikroprozessor (1) geschaltet ist, die so ausgebildet und konfiguriert ist, dass sie einen Ladevorgang zur Aufladung der Akkueinheit (74) steuert.

9. Bodeneinheit nach Anspruch 8, **dadurch gekennzeichnet, dass**
- die Schaltungselektronik (99) eine Ladezustandsmesseinrichtung (2) umfasst, die so ausgelegt ist, dass sie den momentanen Ladezustands einer Akkueinheit (74) misst und ein entsprechendes Signal an den Mikroprozessor (1) abgibt,
- in dem Mikroprozessor (1) eine Tabelle zur Optimierung der Ladezeit abhängig von dem momentanen Ladezustand der Akkueinheit (74) hinterlegt ist, und
- der Mikroprozessor (1) so ausgebildet und konfiguriert ist, dass er einen Laderegler (3) zur Laderegelung der Akkueinheit (74) der abgespeicherten Tabelle entsprechend steuert.

10. Bodeneinheit nach Anspruch 8 oder 9, **dadurch gekennzeichnet, dass** an der Unterseite der Bodeneinheit (80) eine Ladezustandsanzeige (4) vorgesehen ist, die eine oder mehrere LEDs (4a, 4b, 4c) umfasst, und dass die Schaltungselektronik (99) so ausgebildet und konfiguriert ist, dass sie den Ladezustand der Akkueinheit (74) über die Ladezustandsanzeige (4) anzeigt.

11. Bodeneinheit nach Anspruch 10, **dadurch gekennzeichnet, dass** die Schaltungselektronik (99) so ausgebildet und konfiguriert ist, dass sie die Ladezustandsanzeige (4) abschaltet, wenn das untere Kontaktelement (95) mit dem Kontaktelement (107) der Ladestation in Kontakt kommt.

12. Bodeneinheit nach Anspruch 10 oder 11, **dadurch gekennzeichnet, dass** die Schaltungselektronik (99) so ausgebildet und konfiguriert ist, dass sie die Ladezustandsanzeige (4) einschaltet, wenn der Kontakt des unteren Kontaktelements (95) mit dem Kontaktelement (107) der Ladestation unterbrochen wird.

13. Bodeneinheit nach Anspruch 12, **dadurch gekennzeichnet, dass** die Schaltungselektronik (99) so ausgebildet und konfiguriert ist, dass sie die Ladezustandsanzeige (4) nach Ablauf einer vorher bestimmten Zeit nach dem Einschalten wieder ausschaltet.

14. Bodeneinheit nach einem der Ansprüche 8 bis 13, **dadurch gekennzeichnet, dass** die Schaltungselektronik (99) einen Schalter (5) in einer Leiterbahn (106) umfasst, die mit einem Verbraucher (36) verbindbar ist, und der Mikroprozessor (1) so ausgebildet und konfiguriert ist, dass er den Schalter (5) öffnet, wenn das untere Kontaktelement (95) mit dem Kontaktelement (107) der Ladestation in Kontakt kommt, und er den Schalter (5) schließt, wenn der Kontakt zwischen dem unteren Kontaktelement (95) und dem Kontaktelement (107) der Ladestation unterbrochen wird.

15. Batteriegriff für medizinische Diagnostikinstrumente, insbesondere für ein Laryngoskop, **dadurch gekennzeichnet, dass** am unteren Ende der Griffhülse (90) eine Bodeneinheit (80) nach einem der Ansprüche 8 bis 14 angeordnet ist.
